# EUROPÄISCHE PATENTSCHRIFT

(11) **EP 1 175 207 B1**
(45) Veröffentlichungstag und Bekanntmachung des Hinweises auf die Patenterteilung: **25.08.2004**
(21) Anmeldenummer: 00983183.5
(22) Anmeldetag: 29.11.2000
(51) Int. Cl.: A61K 9/48, A61K 9/20

(54) **SPRITZGUSSVERFAHREN FÜR (METH)ACRYLAT-COPOLYMERE MIT TERTIÄREN AMMONIUMGRUPPEN**
INJECTION MOLDING METHOD FOR (METH)ACRYLATE COPOLYMERS HAVING TERTIARY AMMONIUM GROUPS
PROCEDE DE MOULAGE PAR INJECTION POUR COPOLYMERES (METH)ACRYLATE AVEC DES GROUPES AMMONIUM TERTIAIRES

(30) Priorität: 02.12.1999 DE 19958007
(43) Veröffentlichungstag der Anmeldung: 30.01.2002
(73) Patentinhaber: Röhm GmbH, 64293 Darmstadt (DE)
(72) Erfinder: PETEREIT, Hans-Ulrich, 64291 Darmstadt (DE); BECKERT, Thomas, 64297 Darmstadt (DE); ASSMUS, Manfred, 64404 Bickenbach (DE); HÖSS, Werner, 63150 Heusenstamm (DE); FUCHS, Wolfgang, 64665 Alsbach (DE); SCHIKOWSKY, Hartmut, 64285 Darmstadt (DE)
(86) Internationale Anmeldenummer: PCT/EP2000/011922
(87) Internationale Veröffentlichungsnummer: WO 2001/039751

(56) Entgegenhaltungen:
- EP-A- 0 704 207
- EP-A- 0 727 205
- GB-A- 1 298 084
- GB-A- 1 355 324

## Beschreibung

Die Erfindung betrifft ein Verfahren zur Herstellung von Formkörpern mittels Spritzguß, die Formkörper selbst und deren Verwendung für pharmazeutische Zwecke.

### Stand der Technik

US 4 705 695 beschreibt ein Verfahren zum Überziehen von pharmazeutischen Formulierungen mit einem wäßrigen Überzugsmittel, enthaltend ein wasserlösliches (Meth)acrylat-Copolymer mit tertiären Aminogruppen sowie ein wasserunlösliches, neutrales Polymer als Binder. Die Löslichkeit des (Meth)acrylat-Copolymers, bestehend z. B. aus gleichen Anteilen Methylmethacrylat und Dimethylaminoethylmethacrylat, wird durch Einrühren in Pulverform mit Partikelgrößen unter 0,25 mm in Wasser unter gleichzeitiger Zugabe einer Säure bewirkt. Als Binder wird ein unlösliches Copolymer, z. B. aus Methylmethacrylat und Ethylacrylat (70 : 30), eingesetzt. Die Herstellung der Überzugslösung ist relativ aufwendig. Wegen des Gehaltes an Säure hat der Überzug einen unangenehmen Geschmack. Entsprechende Filme lösen sich sowohl in künstlichem Magensaft als auch in Wasser in weniger als zwei Minuten.

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

Im Beispiel werden entsprechende Mischpolymerisate bei 160 °C aufgeschmolzen und nach Zugabe von 6 Gew.-% Glycerinmonostearat gemischt. Die Mischung wird gebrochen und zu einem Pulver vermahlen. Das Pulver wird in die Vorkammer eines Spritzpreßwerkzeugs gefüllt und bei 170 °C unter einem Druck von 150 bar durch eine 0,5 mm weite Öffnung in den Formhohlraum gespritzt. Nach Abkühlung erhält man blasenfreie, leicht opake, dünnwandige Arzneimittelkapseln. Besondere Maßnahmen zur Entfernung niedrig siedender Bestandteile unmittelbar vor der Spritzgußverarbeitung sind nicht offenbart.

GB 1,298,084 beschreibt Kapseln und andere für medizinische Zwecke geeignete Behältnisfornien. Beispielsgemäß wird ein (Meth)acrylatcopolymer aus Butylmethacrylat und Dimethylaminoethylmethacrylat unter Zusatz eines Trennmittels zu Kapseln spritzgegossen. Weichmacher und Trockenstellmittel sind nicht enthalten. Ein Entgasungsschritt wird nicht offenbart.

GB 1,355,324 beschreibt Kapselmaterialien z. B. aus verschiedenen Cellulosen für pharmazeutische Zwecke. Beispielsgemäß können Cellulosemischungen, die Weichmacher und Trennmittel enthalten, in einem Entgasungsextruder zu Granulaten verarbeitet werden. Diese können wiederum zu Kapselmaterialien spritzgegossen werden. Trockenstellmittel sind nicht erwähnt.

EP 0 727 205 A2 betrifft ein thermoplastisches Überzugs- und Bindemittel für Arzneiformen, das z. B. aus verschiedenen (Meth)acrylatcopolymeren, u.a. auch solchen mit tertiären Aminogruppen, bestehen kann, die mit Fließmitteln formuliert werden, die auch Trennmittel oder Weichmacher im Sinne der vorliegenden Erfindung, z. B. Glycerolmonostearat oder Polyethylenglykol 4000, sein können. Die Verarbeitungsmöglichkeit im Spritzgussverfahren wird in allgemeiner Form erwähnt, ist jedoch wegen der Abfall- und Entsorgungsproblematik weniger bevorzugt. Spezielle Maßnahmen für den Spritzguß, wie der Einsatz von Trockenstellmitteln oder ein Entgasungsschritt, sind nicht erwähnt.

### Aufgabe und Lösung

Es wurde als Aufgabe gesehen, ein Verfahren bereitzustellen, daß es erlaubt, die bekannten (Meth)acrylat-Copolymere, die Monomere mit tertiären Aminogruppen enthalten, im Spritzgußverfahren zu verarbeiten. Auf diese Weise sollen Formkörper erhalten werden, die magensaftlösliche Eigenschaften aufweisen und hohen mechanischen Anforderungen genügen und daher z. B. als Kapseln (Steckkapseln), die als Behältnisse für pharmazeutische Wirkstoffe dienen, verwendet werden können.

Verfahren zur Herstellung von Formkörpern mittels Spritzguß
mit den Verfahrensschritten
a) Aufschmelzen eines (Meth)acrylat-Copolymeren, das sich aus 30 bis 80 Gew.-% radikalisch polymerisierten C1- bis C4-Alkylestern der Acryl- oder der Methacrylsäure und 70 bis 20 Gew.-% (Meth)acrylat-Monomeren mit einer tertiären Aminogruppe im Alkylrest zusammensetzt,
   wobei das (Meth)acrylat-Copolymere in Mischung mit 1 bis 70 Gew.-% von einem Weichmacher und einem Trockenstellmittel im Verhältnis 1: 1 bis 1 : 20 vorliegt,
   wobei mindestens 1 Gew.-% Weichmacher bezogen auf das (Meth)acrylat-Copolymere enthalten ist,
   sowie 0,05 bis 5 Gew.-% eines Trennmittels bezogen auf das Copolymere enthalten sind und
   zusätzlich weitere übliche Additive oder Hilfsstoffe und gegebenenfalls ein pharmazeutischer Wirkstoff in der Mischung enthalten sein können und die Mischung vor dem Aufschmelzen einen Gehalt an niedrigsiedenden Bestandteilen mit einem Dampfdruck von mindestens 1,9 bar bei 120°C von über 0,5 Gew.-% aufweist
b) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird
c) Einspritzen der aufgeschmolzenen und entgasten Mischung **mit einem Gehalt an niedrigsiedenden Bestandteilen mit einem Dampfdruck von mindestens 1,9 bar bei 120°C von höchstens 0,5 Gew.-%** in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

Mittels des erfindungsgemäßen Verfahrens sind neue spritzgegossene Formkörper erhältlich, die Anforderungen hinsichtlich hoher mechanischer Festigkeit und hoher Temperaturstabilität genügen.,

### Ausführung der Erfindung

Das erfindungsgemäße Verfahren zur Herstellung von Formkörpern mittels Spritzguß gliedert sich in die Verfahrensschritte a), b) und c).

### Verfahrensschritt a)

Aufschmelzen eines (Meth)acrylat-Copolymeren, das sich aus 30 bis 80 Gew.-% radikalisch polymerisierten C1- bis C4-Alkylestern der Acryl- oder der Methacrylsäure und 70 bis 20 Gew.-% (Meth)acrylat-Monomeren mit einer tertiären **Aminogruppe** im Alkylrest zusammensetzt, wobei das Meth)acrylat-Copolymere in Mischung mit 1 bis 70 Gew.-% von einem Weichmacher und einem Trockenstellmittel im Verhältnis 1: 1 bis 1 : 20, bevorzugt 1 : 1 bis 1 : 10, besonders bevorzugt 1 : 1 bis 1 : 4, wobei mindestens 1 Gew.-% Weichmacher enthalten ist, 0,05 bis 5 Gew.-%, bevorzugt 0,1 bis 3 Gew.-% eines Trennmittels enthalten sind und zusätzlich weitere übliche Additive oder Hilfsstoffe und gegebenenfalls ein pharmazeutischer Wirkstoff in der Mischung enthalten sein können und die Mischung vor dem Aufschmelzen einen Gehalt an niedrigsiedenden Bestandteilen mit einem Dampfdruck von mindestens 1,9 bar bei 120°C von über 0,5 Gew.-% aufweist.

Das Aufschmelzen des Copolymeren, das in Granulatform oder Pulverform vorliegt, erfolgt bevorzugt in einem Extruder bei einer Temperatur von 80 bis 250 °C

### Das (Meth)acrylat-Copolymer

Das (Meth)acrylat-Copolymer setzt sich aus 30 bis 80 Gew.-% radikalisch polymerisierten C₁- bis C₄-Alkylestern der Acryl- oder der Methacrylsäure und 70 bis 20 Gew.-% (Meth)acrylat-Monomeren mit einer tertiären **Aminogruppe** im Alkylrest zusammen.

Geeignete Monomere mit funktionellen tertiären **Aminogruppen** sind in US 4 705 695, Spalte 3, Zeile 64 bis Spalte 4, Zeile 13 aufgeführt. Insbesondere zu nennen sind Dimethylaminoethylacrylat, 2-Dimethylaminopropylacrylat, Dimethylaminopropylmethacrylat, Dimethylaminobenzylacrylat, Dimethylaminobenzylmethacrylat, (3-Dimethylamino-2,2-dimethly)propylacrylat, Dimethylamino-2,2-dimethly)propylmethacrylat, (3-Diethylamino-2,2-dimethly)propylacrylat und Diethylamino-2,2-dimethly)propylmethacrylat. Besonders bevorzugt ist Dimethylaminoethylmethacrylat.

Der Gehalt der Monomere mit tertiären **Aminogruppen** im Copolymeren kann vorteilhafterweise zwischen 20 und 70 Gew.-%, bevorzugt zwischen 40 und 60 Gew.-% liegen. Der Anteile der C₁- bis C₄-Alkylestern der Acryl- oder Methacrylsäure beträgt 80 - 30 Gew.-%. Zu nennen sind Methylmethacrylat, Ethylmethacrylat, Butylmethacrylat, Methylacrylat, Ethylacrylat und Butylacrylat.

Ein geeignetes (Meth)acrylatcopolymer mit tertiären Aminogruppen kann z. B. aus 20 - 30 Gew.-% Methylmethacrylat, 20 - 30 Gew.-% Butylmethacrylat und 60 - 40 Gew.-% Dimethylaminoethylmethacrylat aufgebaut sein.

Ein konkret geeignetes handelsübliches (Meth)acrylatcopolymer mit tertiären Aminogruppen ist z. B. aus 25 Gew.-% Methylmethacrylat, 25 Gew.-% Butylmethacrylat und 50 Gew.-% Dimethylaminoethylmethacrylat aufgebaut (EUDRAGIT® E100).

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

Die mittlere Teilchengröße kann bei Pulvern wie folgt bestimmt werden:
- Durch Luftstrahlsiebung zur einfachen Aufteilung des Mahlproduktes in wenige Fraktionen. Diese Methode ist in diesem Meßbereich etwas ungenauer als die Alternativen. Mindestens 70, bevorzugt 90 % der Teilchen bezogen auf die Masse (Masseverteilung) sollen im Größenbereich von 1 - 40 µm bevorzugt zwischen 5 und 35, insbesondere zwischen 10 und 20 µm liegen.
- Eine gut geeignete Meßmethode ist die Laserbeugung zur Bestimmung der Korngrößenverteilung. Handelsübliche Geräte erlauben die Messung in Luft (Fa. Malvern S3.01 Partikelsizer) oder bevorzugt in flüssigen Medien (Fa. LOT, Galai CIS 1). Voraussetzung für die Messung in Flüssigkeiten ist, das sich das Polymer darin nicht löst oder die Teilchen auf eine andere Weise während der Messung verändern. Ein geeignetes Medium ist z. B. eine stark verdünnte (ca. 0,02%ige) wäßrige Polysorbat 80 Lösung.

### Mischungen

Das (Meth)acrylat-Copolymere liegt in Mischung mit 1 bis 70 Gew.-% eines Weichmachers und eines Trockenstellmittels im Verhältnis 1 : 1 bis 1 : 20, bevorzugt 1 : 1 bis 1 : 10, besonders bevorzugt von 1 : 1 bis 1: 4 vor. Gegebenenfalls kann die Mischung noch weitere pharmazeutisch übliche Hilfsstoffe enthalten z. B. in einem Anteil von 0,001 Gew.-% bis 30 Gew.-%, bezogen auf das (Meth)acrylat-Copolymere, enthalten.

Zur Steuerung der Wirkstoffabgabe kann es im Einzelfall vorteilhaft sein, weitere Polymere zuzumischen. Der Anteil weiterer Polymere an der Mischung beträgt jedoch nicht mehr als 20 Gew.-%, bevorzugt höchstens 10 Gew.-%, insbesondere 0 - 5 Gew.-% .-%, bezogen auf das (Meth)acrylat-Copolymere, beträgt.

Beispiele für solche weiteren Polymere sind: Polyvinylpyrolidone, Polyvinylalkohole, anionische (Meth)acrylat-Copolymere aus Methylmethacrylat und/oder Ethylacrylat und Methacrylsäure (EUDRAGIT® L 100, EUDRAGIT® S 100, EUDRAGIT® L 100-55). Anionische (Meth)acrylat-Copolymere aus Methylmethacrylat, Methylacrylat und Methacrylsäure, Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT® NE 30 D), Copolymere aus Methylmethacrylat und Butylmethacrylat (PLASTOID® B) oder (Meth)acrylat Copolymere mit quaternären Ammoniumgruppen (EUDRAGIT® RL bzw. EUDRAGIT® RS).

Weiterhin können ein oder mehrere pharmazeutische Wirkstoffe enthalten sein, die sich bei Verarbeitungstemperatur nicht zersetzten.

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen und eine ausreichendeStabilität sowie Penetrationsfähigkeit durch die Haut besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (Interna),
Dermatika, Diuretika, Enzyminhibitoren, Enzympräparate und Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

Beispiele für zum Einfüllen in die Formkörper (Kapseln) oder auch für die Einarbeitung in die Formkörper geeignete Wirkstoffe sind: Ranitidin, Simvastatin, Enalapril, Fluoxetin, Amlodipin, Amoxicillin, Sertalin, Nifidipin, Ciprofloxacin, Acycolvir, Lovastatin,Epoetin, Paroxetin, Captopril, Nabumeton, Granisetron, Cimetidin, Ticarcillin, Triamteren, Hydrochlorothiazid, Verapamil, Paracetamol, Morphinderivate, Topotecan oder der pharmazeutisch verwendeten Salze.

Weichmacher: Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 1 und 35, bevorzugt 2 bis 10 Gew.-% .-%, bezogen auf das (Meth)acrylat-Copolymere.

Trockenstellmittel (Antihaftmittel): Trockenstellmittel haben folgende Eigenschaften: sie verfügen über große spezifische Oberflächen, sind chemisch inert, sind gut rieselfähig und feinteilig. Aufgrund dieser Eigenschaften lassen sie sich vorteilhaft in Schmelzen homogen verteilen und erniedrigen die Klebrigkeit von Polymeren, die als funktionelle Gruppen stark polare Comonomere enthalten.

### Beispiele für Trockenstellmittel sind:

Aluminiumoxid, Magnesiumoxid, Kaolin, Talkum, Kieselsäure (Aerosile), Bariumsulfat, Ruß und Cellulose.

### Trennmittel (Formtrennmittel)

Formtrennmittel müssen in einer Menge von von 0,05 Gew-% bis 5, bevorzugt 0,1 bis 3 Gew.-% bezogen auf das Copolymere hinzugefügt werden.

Im Gegensatz zu Trockenstellmitteln haben Formtrennmittel die Eigenschaft, die Klebkraft zwischen dem Formteilen und der Werkzeugoberfläche, in dem das Formteil hergestellt wird, zu reduzieren. Dadurch wird es möglich, Formteile herzustellen, die nicht zerbrochen und geometrisch nicht deformiert sind. Formtrennmittel sind meist teilverträglich oder unverträglich mit den Polymeren, in denen sie besonders wirksam sind. Durch die Teil- bzw Unverträglichkeit tritt beim Einspritzgen der Schmelze in den Formhohlraum eine Migration in die Grenzfläche des Überganges zwischen Werkzeugwandung und Formteil auf.
Damit Formtrennmittel besonders vorteilhaft migrieren können, muss der Schmelzpunkt des Formtrennmittels 20°C bis 100°C unterhalb der Verarbeitungstemperatur des Polymeren liegen.

Beispiele für Trennmittel (Formtrennmittel) sind:
Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc..

Weitere pharmazeutisch übliche Hilfsstoffe: Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Weitere pharamazeutisch übliche Hilfsstoffe können in Mengen von 0,001Gew-% bis 30 Gew.-%, bevorzugt 0,1 bis 10 Gew.-% bezogen auf das Copolymere vorliegen.

### Niedrigsiedende Bestandteile

Das an sich bekannte (Meth)acrylat-Copolymer weist in seiner Handelsform praktisch immer einen Gehalt an niedrigsiedenden Bestandteilen mit einem Dampfdruck von mindestens 1,9 bar bei 120°C von über 0,5 Gew.-% auf. Übliche Gehalte dieser Bestandteile liegen im Bereich von 0,7 bis 2,0 Gew-%. Bei den niedrigsiedenden Bestandteilen handelt es sich in der Hauptsache um Wasser, das aus der Luftfeuchtigkeit aufgenommen wird.

### Verfahrensschritt b)

Entgasen der Mischung bei Temperaturen von mindestens 120 °C, bevorzugt mindestens 150 °C und höchstens 250 °C, wodurch der Gehalt der niedrigsiedenden Bestandteile mit einem Dampfdruck von mindestens 1,9 bar bei 120°C auf höchstens 0,5 Gew.-%, bevorzugt höchstens 0,2 Gew.-% , besonders bevorzugt höchstens 0,1 Gew.-% gesenkt wird. Dadurch kann vermieden werden, daß es während des Spritzgußvorganges in Verfahrensschritt c) zu einer unerwünschten plötzlichen Ausgasung kommt, die zu Blasenbildung oder einem Aufschäumen innerhalb des entstehenden Formkörpers führen würde, der dann unbrauchbar wäre.

Da das (Meth)acrylat-Copolymeren eine Glastemperatur im Bereich von 50 °C aufweist, können niedrigsiedende Bestandteile nicht durch einfaches Trocken bei erhöhter Temperatur entfernt werden, das das Copolymer dabei in ungewünschter Weise versintern oder verfilmen würde.

Deshalb wird der Entgasungsschritt b) bevorzugt durch Extrusionstrocknung mittels eines Extruders mit Entgasungszone oder mittels einer Spritzgießanlage mit einem Spritzgießwerkzeug mit vorgeschalteter Entgasungsöffnung ausgeführt.

Das durch Extrusionstrocknung in einem Extruder mit Entgasungszone erhaltene, entgaste Extrudat kann ohne weitere Verfahrensschritte zur Entfernung siedersiedende Bestandteile unmittelbar auf die Spritzgießmaschine gegeben und direkt zu Formkörpern verarbeitet werden.

Beim Entgasen auf einer Spritzgießanlage mit vorhandener Entgasungsöffnung im Spritzgießzylinder erfolgt die Entgasung vor dem Einpressen der Kunststoffschmelze in die Spritzgießform mittels der genannten Entgasungsöffnung im Spritzgießzylinder.

### Verfahrensschritt c)

Einspritzen der aufgeschmolzenen und entgasten Mischung **mit einem Gehalt an niedrigsiedenden Bestandteilen mit einem Dampfdruck von mindestens 1,9 bar bei 120°C von höchstens 0,5 Gew.-%** in den Formhohlraum eines Spritzgießwierkzeugs, wobei der Formhohlraum eine Temperatur aufweist, die mindestens 10 °C, bevorzugt mindestens 12 °C, besonders bevorzugt mindestens 15 °C, insbesondere mindestens 25 °C oder sogar mindestens 35 °C unterhalb der Glastemperatur des (Meth)acrylat-Copolymeren liegt, Abkühlen der Schmelzemischung und Entnahme des erhaltenen Formkörpers aus der Form.

Die thermoplastische Verarbeitung erfolgt in an sich bekannter Weise mittels einer Spritzgußmaschine bei Temperaturen im Bereich von 80 bis 220 °C, insbesondere zwichen 120 °C und 160°C und bei Drücken von 60 bis 400bar, bevorzugt 80bar bis 120bar.

Die Formtemperatur liegt bei Glastemperaturen der eingesetzten (Meth)acrylat-Copolymeren in Bereich von z. B. 40°C bis 80°C entsprechend niedriger z. B. bei höchstens 30 oder höchstens 20 °C, so daß das Copolymer bereits nach kurzer Zeit nach dem Einspritzvorgang in der Form erstarrt und der fertige Formkörper entnommen bzw. entformt werden kann.

Die Formkörper können aus der Formhöhlung des Spritzgießwerkzeuges ohne Bruch entformt werden und weisen eine gleichmäßige, kompakte einwandfreie Oberfläche auf. Der Formkörper zeichnet sich durch mechanische Belastbarkeit bzw. Elastizität und Bruchfestigkeit aus.

Er weist insbesondere eine Schlagzähigkeit nach ISO 179 gemessen an Probekörpern von mindestens 1,0 kJ/m², bevorzugt von mindestens 1,5 kJ/m², besonders bevorzugt von mindestens 2,0 kJ/m2 auf.

Die Wärmeformbeständigkeit VST (A10), gemessen an Probekörpern nach ISO 306 liegt in etwa zwischen 30°C und 60°C.

Die erfindungsgemäß erhaltenen Formkörper können z. B. die Form einer Kapsel, den Teil einer Kapsel, z. B. einer Kapselhälfte, oder einer Steckkapsel aufweisen, die als Behältnis für einen pharmazeutischen Wirkstoff dient. Es können z. B. in Bindemitteln enthaltene Wirkstoffe in Form von Pellets eingefüllt werden und hiernach werden die beiden Kapselteile durch Kleben, Verschweißen durch Laser, Ultraschall bzw. Mikrowellen oder mittels Schnappverbindung zusammengefügt.

Nach diesem Verfahren können erfindungsgemäß auch Kapseln aus unterschiedlichem Material (z.B. Gelatine, anhydrolysierte Stärke, HPMC oder andere Methacrylate) miteinander kombiniert werden. Der Formkörper kann somit auch Teil einer Dosiereinheit sein.

Auch andere Formen, wie Tabletten- oder Linsengeometrien sind möglich. Hierbei enthält der Compound, der für das Spritzgießen zum Einsatz kommt bereits den pharmazeutischen Wirkstoff. In der endgültigen Form liegt der Wirkstoff möglichst gleichmäßig verteilt in kristalliner (Solid Dispersion) oder gelöster Form (Solid Solution) vor.

### BEISPIELE

### Beispiel 1: Magensaftlöslicher Formkörper / Entgasung im Extruder

Auf einem Doppelschneckenextruder (Leistritz LMS 30.34) wurde ein erfindungsgemäße Mischung (Compound) hergestellt.

Über eine gravimetrische Dosiereinrichtung wurde in die Einzugszone des Doppelschneckenextruders 10 kg Granulat ein Methacrylat-Copolymer aus 25 Gew.-% Methylmethacrylat, 25 Gew.-% Butylmethacrylat und 50Gew.-% Dimethylaminoethylmethacrylat (EUDRAGIT® E100) pro h zudosiert. Über eine weitere gravimetrische Dosiereinrichtung wurde zusätzlich kontinuierlich in die Einzugszone des Doppelschneckenextruders 20 Gew.-% Talkum (Trockenstellmittel) sowie 0,25 Gew-% Stearylalkohol (Formtrennmittel) zudosiert.

Mit einer Schneckendrehzahl von 120U/min wurde die Komponenten in den Extruder eingezogen, das Polymere plastifiziert und das Talkum homogen in die Schmelze eingemischt. Die eingestellte Schmelzetemperatur betrug 160°C. Nach einer Länge von 50% der Gesamtlänge des Doppelschneckenextruders ist in der Zylinderwandung eine Öffnung eingebracht, über die mittels einer Membranpumpe Triethylcitrat in einer Menge von 5 Gew.-% bezogen auf die Polymermenge zugepumpt wird. Nach einer Mischzone für die Homogenisierung der Mischung ist im Schneckenzylinder eine Entgasungsöffnung eingebracht, die eine Öffnung zur Umgebung aufweist. Es kann beobachtet werden, dass aus der Entgasungszone Dampf austritt.

Mittels einer Düse wurden vier Stränge aus dem Extruder abgeformt, über ein gekühltes Blech abgezogen und zu Granulat geschnitten. An erhaltenen Granulat wurde der Feuchtegehalt (bestimmt nach K. Fischer) mit 0,05% ermittelt. Eine Überprüfung des nicht extrudierten Ausgangsgranulats ergab 0,94% Wasser.

Zur Verbesserung der Reiselfähigkeit und Reduzierung der Klebrigkeit wurde das Granulat nach Zusatz von 0,05% Talkum in einer Mischtrommel intensiv gemischt, so dass die Granulatkörner eine gepuderte Oberfläche aufwiesen.

### Spritzgießverarbeitung des erhaltenen Granulats:

Die erhaltene Mischung (Compound) wurde in den Trichter einer Spritzgießmaschine (Arburg Allrounder 221-55-250) gegeben und Formkörper spritzgegossen.

### An der Spritzgießmaschine wurden folgende Temperaturen eingestellt:

Zone 1 (Einzugszone): 70°C, Zone 2: 120°C Zone 3: 160°C Zone 4: 160°C Zone 5 (Düse): 130°C. Einspritzdruck 60 bar, Nachdruck: 50 bar, Staudruck 5bar. Werkzeugtemperatur: 17°C

Als Formkörper wurde ein Plättchen 60 x 45 x 1 mm spritzgegossen.
Es konnten Plättchen frei von Schlieren mit einwandfreier glatter Oberfläche hergestellt werden. Die Plättchen liesen sich problemlos entformen und sind geometriestabil.

### Beispiel 2: (Vergleichsbeispiel)

Wie in Beispiel 1 beschrieben wurde ein Compound hergestellt, wobei die Entgasungsöffnung am Ende des Extruders verschlossen wurde.
Am aus dem Extruder erhaltenen Granulat wurde ein Feuchtegehalt von 0,63% bestimmt.

Das Granulat wurde wie in Beispiel 1 auf die Spritzgießmaschine gegeben und unter Beibehaltung der Parametereinstellung verarbeitet.
Die erhaltenen Formkörper zeigten Schlieren und Oberflächenfehler und entsprechen nicht den Anforderungen.
Nach Herstellung von 7 Formkörpem traten Probleme mit dem Granulateinzug auf der Spritzgießmaschine auf. Es konnte festgestellt werden, dass im Einzugsbereich der Schnecke sich kondensierte Feuchtigkeit angesammelt hatte, die zum Zusammenbrechen der Feststoffförderung führte.

### Beispiel 3: (Vergleichsbeispiel / ohne Trockenstellmittel)

Es wurde wie in Beispiel 1 beschrieben, eine Mischung lediglich unter Zusatz von 0,25 Gew-% Stearylalkohol aber ohne Trockenstellmittel hergestellt. Das erhaltene Granulat wurde wie in Beispiel 1 beschrieben auf die Spritzgießmaschine gegeben und verarbeitet. Es war nicht möglich, ein Formteil herzustellen. Das Plättchen klebt im Spritzgießwerkzeug und kann nicht entformt werden.

### Beispiel 4: Entgasung auf einer Spritzgießmaschine

Eine wie unter Beispiel 1 beschriebene Mischung (Compound) wurde hergestellt, wobei die Entgasungsöffnung verschlossen war. Am erhaltenen Granulat wurde der Feuchtegehalt nach K. Fischer mit 0,57 % ermittelt An der Spritzgußmaschine wurde die Spritzgießeinheit gegen eine Einheit mit einer Entgasungsöffnung im Schneckenzylinder ausgetauscht.
Das Granulat konnte problemlos zu Formkörpern verarbeitet werden.

### Beispiel 5:

Es wurde eine Mischung wie in Beispiel 1 angegeben hergestellt.
Auf einer Spritzgußmaschine (Modell Boy micro 22) wurden mit einem Spritzgießwerkzeug für Kapseln mit einer Länge von 16 mm, einen mittleren Außendurchmesser von 6,8 mm, der sich zum geschlossenen Ende hin auf 4mm verjüngt und eine Wandstärke von 0,6 mm spritzgegossen.

Nach dem Einspritzen der Schmelze und einer Nachdruckzeit von 6 Sekunden wurde nach einer Kühlzeit von 18 Sekunden das Werkzeug geöffnet und die Kapseln entformt. Die Kapseln konnten ohne Bruch aus dem Werkzeug entformt werden. Es wurden mechanisch stabile, opake bis weißlich gefärbte Kapseln erhalten.

### Prüfung der Magensaftlöslichkeit der hergestellten Kapseln

Die hergestellten Kapseln wurden gemäß Pharm. Eur in einem Paddle Gerät mit einer Drehzahl von 100/min hinsichtlich Lösungsverhalten geprüft.
In künstlichem Magensaft (0,1 n Salzsäure, pH 1,2) löst sich die Kapsel nach 2 Stunden auf, in demineralisiertem Wasser war nur eine leicht Quellung verbunden mit einer weißlichen Trübung zu beobachten, während in Phosphatpuffer pH 7,5 nach 2 Stunden keine Veränderung festzustellen war.

## Patentansprüche

1. Verfähren zur Herstellung von Formkörpem mittels Spritzguß
mit den Verfahrensschritten
a) Aufschmelzen eines (Meth)acrylat-Copolymeren, das sich aus 30 bis 80 Gew.-% radikalisch polymerisierten C1- bis C4-Alkylestern der Acryl- oder der Methacrylsäure und 70 bis 20 Gew.-% (Meth)acrylat-Monomeren mit einer tertiären **Aminogruppe** im Alkylrest zusammensetzt,
wobei das (Meth)acrylat-Copolymere in Mischung mit 1 bis 70 Gew.-% von einem Weichmacher und einem Trockenstellmittel im Verhältnis 1:1 bis 1 : 20 vorliegt,
wobei mindestens 1 Gew.-% Weichmacher bezogen auf das (Meth)acrylat-Copolymere enthalten ist,
sowie 0,05 bis 5 Gew.-% bezogen auf das Copolymere eines Trennmittels enthalten sind und
Zusätzlich weitere übliche Additive oder Hilfsstoffe und gegebenenfalls ein pharmazeutischer Wirkstoff in der Mischung enthalten sein können und die Mischung vor dem Aufschmelzen einen Gehalt an niedrigsiedenden Bestandteilen mit einem Dampfdruck von mindestens 1,9 bar bei 120°C von über 0,5 Gew.-% aufweist,
b) Entgasen der Mischung im thermoplastischen Zustand bei Temperaturen von mindestens 120 °C, wodurch der Gehalt der niedrigsiedenden Bestandteile mit einem Dampfdruck von mindestens 1,9 bar bei 120°C auf höchstens 0,5 Gew.-% gesenkt wird
c) Einspritzen der aufgeschmolzenen und entgasten Mischung **mit einem Gehalt an niedrigsiedenden Bestandteilen mit einem Dampfdruck von** **mindestens 1,9 bar bei 120°C von höchstens 0,5 Gew.-%** in den Formhohlraum eines Spritzgießwerkzeugs, wobei der Formhohlraum eine Temperatur aufweist, die mindestens 10 °C unterhalb der Glastemperatur des (Meth)acrylat-Copolymeren liegt, Abkühlen der Schmelzemischung und Entnahme des erhaltenen Formkörpers aus der Form.

2. Verfahren nach Anspruch 1, **dadurch gekennzeichnet, daß** der Entgasungsschritt b) durch Extrusionstrocknung mittels eines Extruders mit Entgasungszone oder mittels einer Spritzgießanlage mit einer, dem Spritzgießwerkzeug vorgeschalteten Entgasungsöffnung im Spritzgießzylinder erfolgt.

3. Spritzgegossener Formkörper herstellbar in einem Verfahren nach Anspruch 1 oder 2.

4. Formkörper nach Anspruch 3, **dadurch gekennzeichnet, daß** er eine Schlagzähigkeit nach ISO 179 von mindestens 1,5 kJ/m² aufweist.

5. Formkörper nach Anspruch 3 oder 4, **dadurch gekennzeichnet, daß** es sich um eine Kapsel, den Teil einer Kapsel oder den Teil einer Dosiereinheit handelt.

6. Formkörper nach Anspruch 3 oder 4, **dadurch gekennzeichnet, daß** er einen pharmazeutischen Wirkstoffe enthält.

7. Verwendung eines Formkörpers nach einem oder mehreren der Ansprüche 3 bis 6 als Behältnis oder als Träger für einen pharmazeutischen Wirkstoff.

## Claims

1. Method of producing moulded parts by injection moulding, comprising the steps of
a) melting a (meth)acrylate copolymer which is made up of 30 to 80 wt.% of radically polymerised C₁₋₄ alkyl esters of acrylic or methacrylic acid and 70 to 20 wt.% of (meth)acrylate monomers with a tertiary amino group in the alkyl moiety,
the (meth)acrylate copolymer being present in admixture with 1 to 70 wt.% of a plasticiser and a drying agent in a ratio of 1 : 1 to 1 : 20,
wherein at least 1 wt.% of plasticiser is present, based on the (meth)acrylate copolymer,
and 0.05 to 5 wt.% of a mould-release agent is present, based on the copolymer, and
other conventional additives or adjuvants and optionally a pharmaceutical active substance may also be present in the mixture and before melting, the mixture contains more than 0.5 wt.% of low-boiling ingredients with a vapour pressure of at least 1.9 bar at 120°C,
b) degassing the mixture in the thermoplastic state at temperatures of at least 120°C, thereby lowering the content of the low-boiling ingredients with a vapour pressure of at least 1.9 bar at 120°C to not more than 0.5 wt.%,
c) injecting the molten, degassed mixture containing at most 0.5 wt.% of low-boiling ingredients with a vapour pressure of at least 1.9 bar at 120°C into the moulding cavity of an injection moulding tool, the moulding cavity being at a temperature which is at least 10°C below the glass transition temperature of the(meth)acrylate copolymer, cooling the molten mixture and removing the cooled moulding from the mould.

2. Method according to claim 1, **characterised in that** the degassing step b) is carried out by extrusion drying using an extruder with a degassing zone or using an injection moulding apparatus having a degassing opening in the injection moulding cylinder, upstream of the injection moulding tool.

3. Injection-moulded part which may be produced by a process according to claim 1 or 2.

4. Moulded part according to claim 3, **characterised in that** it has an impact strength according to ISO 179 of at least 1.5 kJ/m².

5. Moulded part according to claim 3 or 4, **characterised in that** it is a capsule, part of a capsule or part of a dosage unit.

6. Moulded part according to claim 3 or 4, **characterised in that** it contains a pharmaceutical active substance.

7. Use of a moulded part according to one or more of claims 3 to 6 as a container or carrier for a pharmaceutical active substance.

## Revendications

1. Procédé de fabrication de corps moulés par moulage par injection selon les étapes suivantes
a) fusion d'un copolymère (méth)acrylate composé de 30 à 80 % en poids d'esters d'alkyle C1 à C4 radicalement polymérisés d'acides acryliques ou d'acides méthacryliques et de 70 à 20 % en poids de monomère (méth)acrylate avec un groupe amino tertiaire dans le reste alkyle,
le copolymère (méth)acrylate se présentant en mélange avec 1 à 70 % en poids d'un agent plastifiant et d'un modulateur de séchage dans un rapport de 1/1 à 1/20,
et contenant au moins 1 % en poids de l'agent plastifiant par rapport au copolymère (méth)acrylate, ainsi que
0,05 à 5 % en poids d'un agent de séparation par rapport au copolymère, et
d'autres additifs ou adjuvants usuels avec éventuellement un agent pharmaceutique pouvant en outre être contenus dans le mélange, et le mélange présentant, avant la fusion, une teneur de plus de 0,5 % en poids de composants à bas point d'ébullition avec une pression de la vapeur d'au moins 1,9 bar à 120°C,
b) dégazage du mélange à l'état thermoplastique à des températures d'au moins 120°C, la teneur des composants à bas point d'ébullition avec une pression de la vapeur d'au moins 1,9 bar à 120°C étant ainsi abaissée à 0,5 % en poids maximum,
c) moulage par injection du mélange fondu et dégazé avec une teneur en composants à bas point d'ébullition avec une pression de la vapeur d'au moins 1,9 bar à 120°C à 0,5 % en poids maximum dans l'empreinte d'un moule pour injection, l'empreinte présentant une température située au moins à 10°C sous la température de transition vitreuse du copolymère (méth)acrylate, refroidissement du mélange fondu et prélèvement du corps moulé obtenu hors du moule.

2. Procédé selon la revendication 1,
**caractérisé en ce que**
l'étape de dégazage b) est réalisée par séchage par extrusion au moyen d'une extrudeuse ayant une zone de dégazage ou à l'aide d'une installation de moulage par injection ayant un orifice de dégazage placé en amont du moule pour injection dans le piston de moulage par injection.

3. Corps moulé par moulage par injection pouvant être fabriqué par un procédé selon la revendication 1 ou 2.

4. Corps moulé selon la revendication 3,
**caractérisé en ce qu'**
il présente une résilience d'au moins 1,5 kJ/m² selon la norme ISO 179.

5. Corps moulé selon la revendication 3 ou 4,
**caractérisé en ce qu'**
il s'agit d'une capsule, de la partie d'une capsule ou de la partie d'une unité de dosage.

6. Corps moulé selon la revendication 3 ou 4,
**caractérisé en ce qu'**
il contient des agents pharmaceutiques.

7. Utilisation d'un corps moulé selon l'une ou plusieurs des revendications 3 à 6,0 comme récipient ou comme support d'un agent pharmaceutique.
